(19) Europäisches Patentamt
European Patent Office
Office européen des brevets

(11) **EP 0 505 270 B1**

(12) **FASCICULE DE BREVET EUROPEEN**

(45) Date de publication et mention
de la délivrance du brevet:
**31.01.1996 Bulletin 1996/05**

(51) Int. Cl.$^6$: **A61M 1/10**, **A61M 1/12**

(21) Numéro de dépôt: **92400719.8**

(22) Date de dépôt: **18.03.1992**

(54) **Pompe sanguine implantable**

Implantierbare Blutpumpe

Implantable blood pump

(84) Etats contractants désignés:
**AT BE CH DE DK ES FR GB GR IT LI LU NL SE**

(30) Priorité: **20.03.1991 FR 9103386**

(43) Date de publication de la demande:
**23.09.1992 Bulletin 1992/39**

(73) Titulaires:
- **CLINIQUE DE LA RESIDENCE DU PARC**
  **F-13362 Marseille Cedex 10 (FR)**
- **Pol, Vincent**
  **F-13008 Marseille (FR)**

- **Dumas, Jean-Claude**
  **F-13390 Auriol (FR)**

(72) Inventeurs:
- **Pol, Vincent**
  **F-13008 Marseille (FR)**
- **Dumas, Jean-Claude**
  **F-13390 Auriol (FR)**

(74) Mandataire: **Martin, Jean-Jacques et al**
**F-75116 Paris (FR)**

(56) Documents cités:
**EP-A- 0 148 661**        **US-A- 4 750 903**
**US-A- 4 976 729**

## Description

L'invention concerne une pompe sanguine implantable, et plus particulièrement une pompe du type comportant une coque extérieure, au moins une poche fermée essentiellement plate et déformable, ladite poche étant disposée dans la coque et étant reliée à une valve d'entrée pour son remplissage par le fluide sanguin et une valve de sortie pour le refoulement du fluide sanguin, et des moyens motorisés pour comprimer la poche fermée selon une séquence déterminée en lui appliquant une contrainte extérieure.

Il s'agit donc d'une pompe de type péristaltique, dont la conception est choisie pour que cette pompe présente, du point de vue physiologique, un comportement proche de celui de la nature afin d'éviter tout phénomène d'hémolyse (destruction des globules rouges), et de réduire autant que faire se peut les phénomènes de chocs, dus à de brusques variations de pression, dans le fluide sanguin. La conception de la pompe doit être également choisie, dans la mesure du possible, pour que son fonctionnement soit essentiellement non-volumétrique, afin d'écarter les risques de collapsus veineux.

Il existe déjà plusieurs systèmes proposés pour réaliser une prothèse ventriculaire cardiaque.

Parmi les systèmes implantables les plus récents, on peut tout d'abord citer le système comportant une poche plate fermée dont chacune des deux parois est au contact d'un plateau presseur, de façon à comprimer la poche pour le refoulement du fluide sanguin. Ces deux plateaux sont articulés sur l'extrémité libre de deux leviers qui s'écartent ou se rapprochent à la manière de branches de ciseaux, l'autre extrémité des deux leviers étant articulée sur un support commun, et chaque levier supportant, au voisinage de ce support commun, un solénoïde respectif.

Cet ensemble est en particulier décrit dans l'Article "IMPLANTABLE LVAD" publié dans l'ouvrage "Assisted Circulation 2" Springer-Verlag - 1984, pages 115 à 141, au nom de P. M. PORTNER et Collaborateurs.

L'ensemble, constitué par la poche fermée et les moyens motorisés servant à comprimer cette poche, est disposé à l'intérieur d'une coque extérieure, essentiellement rigide, destinée à assurer à la fois le confort de l'implantation et la protection des constituants de la prothèse.

Cependant, le choix d'une coque extérieure rigide et étanche induit des perturbations dans le fonctionnement péristaltique de la pompe, perturbations qui peuvent avoir des conséquences extrêmement graves pour le patient puisqu'elles risquent d'entraîner un collapsus veineux.

Ceci résulte du fait que les déplacements de volumes sanguins dans la poche fermée engendrent des variations de pression dans la coque rigide et étanche, de sorte que la dépression à la fin de la phase d'éjection (refoulement du fluide sanguin) peut atteindre une valeur très élevée.

A titre indicatif, avec une pompe comportant une coque de 300 cm$^3$ et une poche fermée capable d'éjecter 40 cm$^3$ de fluide sanguin par systole, la poche est soumise à la fin de la phase d'éjection à une dépression (comparable à la pression d'éjection) dépassant 70 mm de mercure (soit 9 100 Pa) et pouvant même atteindre 100 mm de mercure (soit 13 000 Pa), ce qui est une valeur très élevée si l'on se rappelle que la pression moyenne d'éjection d'un coeur naturel est de l'ordre de 150 mm de mercure (soit 19 500 Pa).

Un tel système, dont le fonctionnement est de plus sensiblement volumétrique, ne permet pas d'éviter ce phénomène physique de dépression, à moins naturellement de prévoir une coque de volume très élevé, mais alors la pompe présenterait un encombrement qui deviendrait rédhibitoire.

Les brevets US-A-4 976 729 (Holfert et al.) et US-A-4 750 903 (Cheng) présentent des systèmes pneumatiques dont le fonctionnement n'est pas ou peu perturbé par la dépression de coque. Cette dépression est totalement masquée par le gaz de compression, ou le vide d'aspiration qui pilote la membrane ou la poche. Par contre, le bilan énergie de commande sera augmenté pour compenser l'évacuation du sang. Cette augmentation n'est pas un problème pour des systèmes dont la commande est extérieure au malade, et dont le bilan énergétique est de toute manière très mauvais.

La demanderesse a quant à elle proposé un système implantable à fonctionnement non-volumétrique, comme cela est décrit en détail dans le brevet européen N° 0 148 661.

Il s'agit d'une pompe sanguine implantable, du type comportant une coque extérieure, au moins une poche fermée essentiellement plate et déformable, ladite poche étant disposée dans la coque en contactant par l'une de ses parois la surface intérieure de ladite coque et étant reliée à une valve d'entrée pour son remplissage par le fluide sanguin et une valve de sortie pour le refoulement du fluide sanguin, un tambour cylindrique sur une génératrice duquel est fixé un bord d'extrémité de la poche et agencé pour rouler sur l'autre paroi de ladite poche, un étrier en forme de C monté à rotation sur l'axe dudit tambour et dont la branche centrale est reliée, par un élément souple inextensible, à l'autre bord d'extrémité de la poche, et un moteur électrique intégré dans le tambour dont le stator est solidaire dudit tambour et dont le rotor entraîne ledit étrier, ledit moteur permettant d'entraîner en rotation relative et antagoniste l'étrier et le tambour, sur une fraction de tour, pour comprimer la poche qui s'enroule sur ledit tambour afin de refouler le fluide sanguin.

La coque extérieure de la pompe implantable décrite dans le brevet européen précité est en matériau biocompatible (caoutchouc silicone ou polyuréthane par exemple) permettant d'associer à la forme géométriquement déformable de la coque un matériau présentant une mémoire propre (le choix d'un matériau à mémoire géométrique propre améliore en effet la restitution d'énergie, car la mémoire propre du matériau s'ajoute à la déformation résultant du déplacement relatif du tambour et de l'étrier en forme de C).

L'utilisation d'une coque extérieure souple permet certes d'éviter le phénomène de dépression exposé plus haut, mais présente dans la pratique certains inconvénients : en effet, les mouvements des éléments motorisés ne sont pas toujours bien supportés par les organes voisins, d'où une source d'inconfort pour le patient ; de plus, même si la bio-compatibilité est parfaite, on observe la formation à terme d'un néo-tissu rigide à l'extérieur de la coque, d'où il résulte une sensibilité croissante à la dépression lors de la phase d'éjection. Par ailleurs, si la coque extérieure est trop souple, elle peut s'afaisser lors des mouvements des éléments motorisés, et à la limite bloquer l'étrier.

Il serait naturellement séduisant de conserver l'agencement structurel de cette pompe (afin de garder un fonctionnement non-volumétrique), en utilisant une coque extérieure rigide et hermétique (afin d'assurer à la fois le confort de l'implantation et la protection des constituants de la prothèse).

Cependant, on se heurte alors au phénomène physique de dépression déjà mentionné pour le système précédent, générant sur la poche fermée une force d'aspiration lors de la phase d'éjection.

Quel que soit le type de système implantable retenu, le phénomène de dépression a des conséquences multiples :

.    diminution importante du volume de fluide sanguin éjecté ;
.    baisse de pression vers l'aorte ;
.    surconsommation du système de motorisation qui doit vaincre cette force supplémentaire.

Si l'on veut combattre ce phénomène physique, il est nécessaire d'assortir la pompe implantable d'un système de compensation.

Plusieurs systèmes de compensation peuvent être alors envisagés, dont certains ont été effectivement expérimentés : il peut s'agir d'une compensation interne rigide, d'une compensation interne souple, ou encore d'une compensation externe.

Cependant, les explications qui vont suivre montreront que ces systèmes ne donnent pas véritablement satisfaction.

Les système connus de compensation interne rigide comportent des ressorts tarés ou des électro-aimants avec des pistons destinés à chasser, au bon moment, un volume qui permet de compenser la dépression de la coque.

Des systèmes de ce type ont été expérimentés, mais ne sont pas réellement opérationnels à l'heure actuelle. En effet, outre leur encombrement important, ils présentent l'inconvénient de nécessiter un couplage mécanique ou pneumatique entre la mesure de dépression de la coque et le volume de compensation qu'il faut placer dans le thorax du malade. De plus, de tels systèmes ne sont pas exempts de dangers si l'on considère que le moindre passage en pression positive (surcompensation) conduit à interdire le remplissage de la poche.

Les systèmes connus de compensation interne souple sont basés sur le principe d'une poche souple spéciale intra-corporelle, dont le volume est nécessairement important. De tels systèmes ne conviennent pas, car cette poche spéciale devient rapidement le siège d'une formation de néo-tissus rigides : la poche spéciale de compensation perd alors progressivement sa capacité de déformation, et finit par devenir un volume mort supplémentaire, comme celui de la coque. De toute façon, la compensation réelle que l'on pouvait espérer était en fait dès le départ très limitée, en raison de l'équilibre qui s'établit rapidement (on est en effet en présence d'un système qui se comporte comme un système fermé).

Il reste alors la solution d'une compensation externe qui reste théoriquement possible : en effet, si la coque étanche reste mise à la pression atmosphérique par un tube permettant sa "respiration", le système retrouve alors les performances qu'il possède en coque ouverte. Cependant, cette solution implique d'utiliser un cathéter transcutané qui constitue une voie d'infection, et ce surtout s'il s'agit d'une implantation à long terme. De plus, si l'implantation est complètement interne, y compris sa source d'énergie, un tel cathéter devient vite insupportable pour le malade.

On a essayé en variante d'utiliser un cathéter avec une seringue pour créer une légère dépression, mais cette solution n'est guère plus satisfaisante dans la pratique.

Finalement, les systèmes connus de compensation ne permettent pas de combattre de manière satisfaisante le phénomène physique de dépression lorsqu'une coque rigide et étanche est utilisée, de sorte que les solutions actuelles restent limitées à des compromis de ces systèmes, sans progrès déterminant.

Il est intéressant de constater que la plupart des spécialistes, devant l'obligation de prévoir un système de compensation, se sont attachés à réduire le volume mort de la coque. Il apparaît cependant que cette approche ne convient pas, car, si le volume de la coque est divisé par deux, la dépression est sensiblement doublée lors de l'éjection, de sorte que la force qui s'applique sur le système de motorisation devient très importante, et de plus toutes les parties libres de la poche souple se mettent à gonfler en diminuant d'autant le volume éjecté. Il est clair que ces zones libres sont impossibles à éviter, surtout dans les parties qui réunissent la poche souple aux valves d'admission ou d'éjection. Avec une pompe à plateaux presseurs du type précité, on parvient peut-être à réduire les surfaces libres de la poche souple, mais les forces qui s'appliquent sur les plateaux presseurs atteignent rapidement plusieurs kilos, paralysant ainsi le fonctionnement de la pompe, à moins naturellement de prévoir des énergies très importantes.

L'invention a pour objet de réaliser une pompe sanguine implantable plus performante, dont la structure permette d'avoir à la fois un fonctionnement non-volumétrique, et sans avoir à prévoir un système supplémentaire de compensation.

Il s'agit plus particulièrement d'une pompe sanguine implantable, comportant une coque extérieure, au moins une poche fermée essentiellement plate et déformable, ladite poche étant disposée dans la coque en contactant par l'une de ses parois la surface intérieure de ladite coque et étant reliée à une valve d'entrée pour son remplissage par le fluide sanguin et une valve de sortie pour le refoulement du fluide sanguin, un tambour cylindrique sur une génératrice duquel est fixé un bord d'extrémité de la poche et agencé pour rouler sur l'autre paroi de ladite poche, un étrier en forme de C monté à rotation sur l'axe dudit tambour et dont la branche centrale est reliée, par un élément souple inextensible, à l'autre bord d'extrémité de la poche, et un moteur électrique intégré dans le tambour dont le stator est solidaire dudit tambour et dont le rotor entraîne ledit étrier, ledit moteur permettant d'entraîner en rotation relative et antagoniste l'étrier et le tambour, sur une fraction de tour, pour comprimer la poche qui s'enroule sur ledit tambour afin de refouler le fluide sanguin, caractérisée par le fait que la coque extérieure est essentiellement rigide et constitue un boîtier étanche dans lequel la poche fermée peut se déformer, et par le fait que la poche fermée est au moins partiellement réalisée en un matériau essentiellement inextensible dans deux directions, de façon que le volume intérieur d'origine de la poche reste constant quelle que soit sa forme, ladite poche étant ainsi insensibilisée à une dépression extérieure tendant à augmenter son volume intérieur lors du refoulement du fluide sanguin.

Selon un mode de réalisation particulièrement avantageux, le matériau essentiellement inextensible est un composite constitué d'une couche de polymère et de deux nappes croisées de fibres intimement liées au polymère.

De préférence alors, les deux nappes croisées de fibres sont noyées dans la couche de polymère ; en variante, les deux nappes croisées de fibres constituent une trame collée extérieurement sur la surface de la couche de polymère, ou encore intérieurement sur la surface de la couche de polymère.

Avantageusement encore, les deux nappes croisées de fibres sont organisées selon deux directions sensiblement orthogonales et/ou sont orientées diagonalement par rapport à une direction parallèle à la direction générale de l'axe du tambour. En particulier, les deux nappes croisées de fibres sont disposées symétriquement par rapport à ladite direction parallèle à l'axe du tambour.

De préférence, la couche de polymère est réalisée en silicone ou en polyuréthane.

De préférence également, les fibres constitutives de l'une au moins des nappes croisées sont des fibres synthétiques, ou des fibres de verre, ou encore des fibres de carbone.

Il est également avantageux que le matériau essentiellement inextensible concerne au moins la partie des parois de la poche qui ne reste pas en contact avec la surface intérieure de la coque.

De préférence encore, la poche constitue un ensemble moulé en une seule pièce.

Avantageusement enfin, la coque extérieure essentiellement rigide est réalisée à partir de fibres de carbone.

D'autres caractéristiques et avantages de l'invention apparaîtront plus clairement à la lumière de la description qui va suivre et des dessins annexés, concernant un mode de réalisation particulier, en référence aux figures où :

- les figures 1 à 3 sont des vues, avec arrachement partiel, d'une pompe implantable conforme à l'invention ;
- les figures 4a à 4c sont des vues en coupe schématique, prises selon la ligne IV-IV de la figure 2, illustrant le fonctionnement de cette pompe implantable, et montrant les zones de la poche fermée qui sont soumises à une force d'aspiration lors de la phase d'éjection (figure 4c) ;
- la figure 5 est une vue à plus grande échelle du détail X des figures 1 à 3, concernant la structure propre de la poche fermée, et permettant de distinguer deux nappes croisées de fibres, ici noyées dans la couche de polymère ;
- la figure 6 est une coupe selon VI-VI de la figure 5 ;
- les figures 7 et 8 sont des coupes analogues à celle de la figure 6, illustrant des variantes dans lesquelles les fibres constituent une trame collée respectivement extérieurement ou intérieurement sur la surface de la couche de polymère.

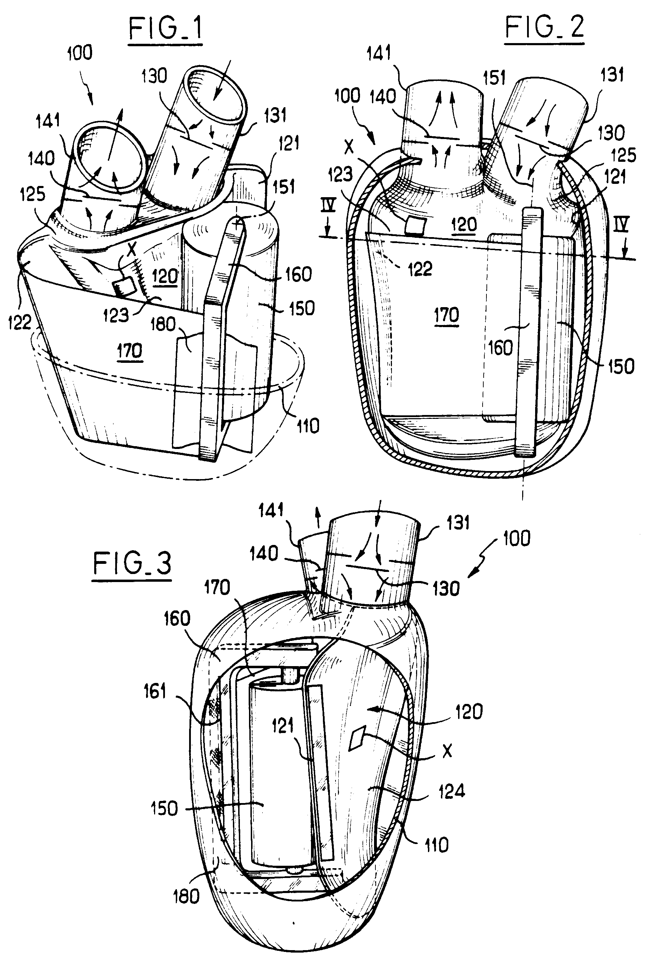

On distingue sur les figures 1 à 3 une pompe sanguine implantable 100 dont la structure générale est conforme à celle qui est décrite dans le brevet européen N° 0 148 661 de la demanderesse, incorporé ici à titre de référence.

La pompe décrite est prévue pour une assistance ventriculaire, destinée à remédier temporairement ou définitivement aux défaillances du ventricule gauche. Il va de soi cependant qu'une telle pompe pourra être aisément modifiée pour réaliser une prothèse cardiaque totale, en prévoyant deux ventricules associés pourvus chacun de leurs valvules propres.

Il s'agit d'une pompe péristaltique et à fonctionnement non-volumétrique, de sorte qu'elle fonctionne avec une admission sanguine sous pression nulle ou faiblement négative, ce qui permet d'éviter tout risque de collapsus en cas de retour veineux insuffisant, et de plus l'absence d'organe mobile à l'intérieur de la poche garantit une très faible hémolyse.

La pompe sanguine implantable 100 comporte une coque extérieure 110, dans laquelle est disposée une poche fermée 120 essentiellement plate et déformable. Cette poche est constituée par deux parois 123, 124 réunies au niveau de bords d'extrémité 121 et 122, et se prolonge supérieurement par une portion supérieure bombée 125 sur laquelle sont montées deux tubulures 131 et 141, contenant respectivement une valve d'entrée 130 et une valve de sortie 140 de type classique (disque basculant par exemple). L'espace intérieur de la poche fermée 110 est ainsi relié à la valve d'entrée 130 pour son remplissage par le fluide sanguin, et à la valve de sortie 140 pour le refoulement du fluide sanguin. La poche fermée 120 contacte par sa paroi dite externe 124 la surface intérieure de la coque 110, et il pourra être avantageux de prévoir une fixation par un point de collage pour limiter le débattement du système pendant le fonctionnement, en évitant des mouvements erratiques à l'intérieur de la coque 110.

Ainsi que cela sera décrit en détail plus loin, la coque extérieure 110 n'est pas souple, ni souple et renforcée par un treillis métallique, comme c'est le cas dans le brevet européen précité, mais est essentiellement rigide de façon à constituer un boîtier étanche dans lequel la poche fermée 120 peut se déformer. On verra alors qu'une structure spéciale est prévue pour la poche 120, cette structure permettant d'éviter d'avoir à prévoir un quelconque système de compensation pour combattre le phénomène de dépression précité qui génère, dans le cas d'une coque extérieure rigide, une force d'aspiration sur la poche fermée lors de la phase d'éjection (refoulement du fluide sanguin).

La pompe implantable 100 comporte également un tambour cylindrique 150 sur une génératrice duquel est fixé un bord d'extrémité (ici le bord 121) de la poche 120, ce tambour étant agencé pour rouler sur la paroi dite interne 123 de ladite poche. Un étrier 160 en forme de C est en outre prévu, articulé autour de l'axe 151 du tambour 150, et dont la branche centrale est reliée, par un élément souple inextensible 170 (ici sous la forme d'un voile) à l'autre bord d'extrémité (ici le bord 122) de la poche fermée 120.

Dans le tambour 150 est intégré un moteur électrique, dont le stator est solidaire dudit tambour, et dont le rotor entraîne l'étrier 160, ledit moteur permettant d'entraîner en rotation relative et antagoniste, sur une fraction de tour, l'étrier 160 et le tambour 150, pour comprimer la poche 120 qui s'enroule sur ledit tambour afin de refouler le fluide sanguin.

Il est à noter que les tubulures 131 et 141 peuvent également être agencées latéralement (variante non représentée ici), cette disposition étant favorable dans la mesure où elle va dans le sens de la poussée péristaltique et où elle induit moins de contraintes sur la poche fermée lors du déplacement du tambour sur la paroi interne de celle-ci.

On distingue par ailleurs, sur les figures 1 et 3, la présence d'un petit voile de liaison 180 passant dans une fente 161 de la branche centrale de l'étrier en forme de C 160, les bords de ce voile étant fixés, par exemple par collage, à la surface intérieure de la coque 110. Un tel voile de liaison peut favoriser une bonne homogénéité du couplage coque / étrier, mais il n'est cependant nullement indispensable pour le fonctionnement de la pompe dont le montage est intrinsèquement "flottant", c'est-à-dire capable de fonctionner sans point d'appui extérieur (de la même façon qu'une poche contractile naturelle), et ce d'autant plus que la coque extérieure 110 est en l'espèce essentiellement rigide, et donc assure pleinement sa fonction de protection contre des sollicitations extérieures (compressions thoraciques par exemple).

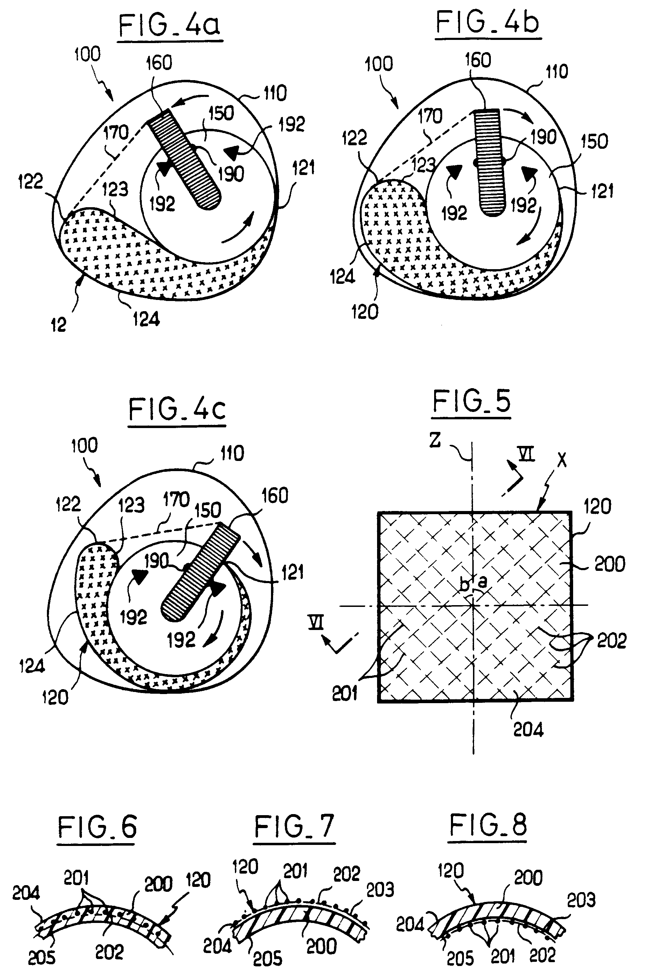

Les figures 4a, 4b et 4c illustrent schématiquement le fonctionnement de la pompe implantable 100.

On a représenté sur cette figure un capteur magnétique 190 disposé sur l'étrier 160, ce capteur servant à détecter la présence d'aimants 191, 192 portés par le tambour 150 et disposés pour correspondre aux deux positions extrêmes de la fraction de tour pour la rotation relative et antagoniste. Il va de soi cependant qu'il ne s'agit que d'un exemple, et que tout autre moyen équivalent de repérage angulaire, par exemple électronique, pourra être utilisé.

La figure 4a correspond à la phase de remplissage (diastole). Lors de cette phase, l'étrier 160 s'abaisse vers la gauche (pour la figure) et le voile 170 se détend, tandis que le stator du moteur (tambour 150) recule sur la paroi interne 123 de la poche 120. Lorsque l'étrier 160 arrive au niveau de la butée magnétique, le sens de rotation du moteur s'inverse.

Commence alors la phase de compression, et la figure 4b correspond à une situation intermédiaire lors de cette phase de compression. Le tambour 150 roule sur la paroi interne 123 de la poche 120 en prenant appui sur l'étrier 160 qui bouge relativement peu. La poche 120 se comprime, et la résistance sur le moteur devient importante et bloque le déplacement du tambour 150. Par la suite, le tambour 150 et l'étrier 160 suivent un mouvement de moindre résistance, réalisant à tout instant une auto-adaptation de façon à équilibrer d'une part la réaction de compression de la poche 120 par le tambour 150, et d'autre part la réaction de traction par le voile 170.

La figure 4c correspond à la phase d'éjection (systole). Lors de cette phase, l'étrier 160 solidaire du rotor du moteur se déplace plus vite que dans la phase de remplissage, et inverse le bras de levier. Le stator (tambour 150) est poussé contre la poche 120, aidant ainsi l'étrier 160 à vider ladite poche (pas tout à fait totalement pour éviter un phénomène d'hémolyse et protéger les globules rouges).

Il convient de noter que le déplacement absolu du tambour 150 dans la coque 110 est en réalité très faible, car c'est principalement la poche 120 qu'on enroule sur ce tambour (soit par enroulement direct, soit en ramenant le bord d'extrémité 122 vers le tambour), à la différence des pompes péristaltiques classiques utilisées dans les circulations extracorporelles où c'est la cavité déformable qui est immobile.

La phase de remplissage se fait alors par retour de l'ensemble à la position initiale, ce retour résultant à la fois de la pression veineuse, de l'élasticité propre de la poche, et d'une commande appliquée au moteur pour assister cette phase de retour.

La figure 4c permet de comprendre aisément que si la coque extérieure 110 est rigide et constitue un boîtier étanche, les zones libres de la poche fermée 120 qui ne sont pas au contact de la surface extérieure du tambour ou de la surface intérieure de la coque rigide 110, c'est-à-dire la presque totalité de la paroi externe 124 et la zone d'extrémité de la paroi interne 123 laissée découverte par le tambour 150, sont soumises à une force d'aspiration lors de la phase d'éjection. Cette force de dépression est certes plus faible que celle que l'on rencontre avec une pompe à plateaux presseurs du type cité plus haut, car la contrainte due à la dépression ne s'applique progressivement que sur une surface de la poche qui devient relativement petite en fin d'éjection, mais cette force existe tout de même, avec les inconvénients que l'on sait pour le patient, et doit de ce fait être combattue.

C'est là qu'intervient une caractéristique essentielle de l'invention, selon laquelle la poche fermée 120 est au moins partiellement (c'est-à-dire au moins pour ses zones libres) essentiellement inextensible dans deux directions, de façon que le volume intérieur d'origine de la poche 120 reste constant quelle que soit sa forme, ladite poche étant ainsi insensibilisée à une dépression intérieure tendant à augmenter son volume intérieur lors du refoulement du fluide sanguin.

La figure 4c permet de bien comprendre cette notion de volume intérieur d'origine constant : en effet, ceci revient à assurer un périmètre constant (en coupe transversale) à la poche 120 (périmètre de la courbe fermée définie en coupe par ladite poche).

Grâce à cette mesure, on parvient à lutter efficacement, et très simplement, contre le phénomène d'aspiration : en évitant tout gonflement indésirable de la poche, notamment dans sa zone critique située entre son raccordement à l'élément souple inextensible et le tambour, on est alors tout à fait sûr que la poche fermée ne peut pas augmenter son volume par suite d'un effet de dépression. Il est clair qu'en l'absence de cette structure particulière, la poche se déformerait à tout le moins dans la zone critique précitée, ce qui augmenterait le volume résiduel qui correspond à un volume non éjecté, et diminuerait de ce fait le débit de la pompe, avec les conséquences graves qui en découleraient pour le patient.

Selon un mode de réalisation préférentiel, le matériau essentiellement inextensible constituant la poche fermée 120, ou à tout le moins les zones de celle-ci qui sont libres lors de la phase d'éjection, est un composite constitué d'une couche de polymère et de deux nappes croisées de fibres intimement liées au polymère.

La figure 5 et la coupe associée de la figure 6 illustrent précisément un fragment X de la poche fermée 120, qui est réalisé conformément à cette structure particulière.

On distingue ainsi une couche 200 de polymère et deux nappes croisées de fibres 201, 202 intimement liées au polymère.

Le polmère peut être du silicone ou du polyuréthane.

Pour ce qui est des fibres constituant les nappes croisées, on pourra choisir des fibres textiles dont la résistance à la traction est suffisamment élevée (par exemple des fibres en matériau commercialisé sous la marque Dacron ou en polytétrafluoréthylène), ou des fibres de verrre ou de carbone, ou encore des fibres en matériau commercialisé sous la marque Kevlar .

Les fibres 201, 202 sont agencées selon deux nappes croisées formant respectivement un angle $\underline{a}$ et $\underline{b}$ avec une direction de référence Z, laquelle est de préférence choisie parallèle à la direction générale de l'axe 151 du tambour 150.

Les deux nappes croisées 201, 202 sont avantageusement organisées selon deux directions orthogonales ($\underline{a} + \underline{b} = 90$ ), diagonalement par rapport à la direction Z, et de préférence symétriquement par rapport à cette direction Z.

Ces deux nappes croisées, 201, 202 peuvent ainsi constituer une trame résistante noyée dans la couche de polymère 200, ainsi que cela est illustré sur la figure 6.

En variante, cette trame peut être collée extérieurement sur la surface 204 de la couche de polymère (figure 7), ou encore intérieurement sur la surface 205 de ladite couche (figure 8) ; cette deuxième solution apparaît toutefois moins performante, car elle risque de casser des globules rouges (risque d'hémolyse). L'état de surface est égalisé par l'utilisation d'une colle adéquate formant une pellicule de liaison 203 : on utilisera de préférence une colle de silicone.

Il sera dans la pratique intéressant de réaliser la poche par moulage, de façon que celle-ci constitue un ensemble moulé en une seule pièce. On s'arrangera naturellement pour que la poche présente un minimum de surface libre en fin de compresion, en prévoyant une poche dont la surface est inférieure à celle du système de compression du volume péristaltique.

Des matériaux approchants, mais à une seule nappe de fibres parallèles, ont déjà été utilisés avec succès pour leur résistance mécanique dans le domaine médical, par exemple pour constituer un péritoine artificiel : cependant, pour cette application particulière, le propos était seulement de rechercher une résistance mécanique élevée pour supporter le poids des organes. Toutefois, de tels matériaux à simple ou double trame, mais à une seule direction de fibres, sont naturellement déformables dans toutes les directions autres que celle des fibres, de sorte qu'ils ne permettent pas de "bloquer" l'élasticité vers l'extérieur, pour préserver un "périmètre constant" sur une courbe fermée, ce qui est normal compte tenu de ce que le problème ne se posait pas dans le cadre d'un péritoine artificiel.

Pour ce qui est de la coque extérieure rigide formant boîtier étanche, on utilisera de préférence une structure en fibres de carbone.

L'invention procure des avantages très importants, car elle permet d'éviter d'avoir à prévoir un quelconque système rapporté de compensation, tout en gardant les avantages d'une coque rigide hermétique, et sans pour autant entraîner une augmentation significative de la puissance électrique nécessaire au fonctionnement de la pompe.

L'invention n'est pas limitée au mode de réalisation qui vient d'être décrit, mais englobe au contraire toute variante reprenant, avec des moyens équivalents, les caractéristiques essentielles énoncées plus haut.

**Revendications**

1. Pompe sanguine implantable (100), comportant une coque extérieure (110), au moins une poche fermée (120) essentiellement plate et déformable, ladite poche étant disposée dans la coque (110) en contactant par l'une (124) de ses parois la surface intérieure de ladite coque et étant reliée à une valve d'entrée (130) pour son remplissage par le fluide sanguin et une valve de sortie (140) pour le refoulement du fluide sanguin, un tambour cylindrique (150) sur une génératrice duquel est fixé un bord d'extrémité (121) de la poche (120) et agencé pour rouler sur l'autre paroi (123) de ladite poche, un étrier (160) en forme de C monté à rotation sur l'axe (151) dudit tambour et dont la branche centrale est reliée, par un élément souple inextensible (172), à l'autre bord d'extrémité (122) de la poche (120), et un moteur électrique intégré dans le tambour (150) dont le stator est solidaire dudit tambour et dont le rotor entraîne ledit étrier, ledit moteur permettant d'entraîner en rotation relative et antagoniste l'étrier (160) et le tambour (150), sur une fraction de tour, pour comprimer la poche (120) qui s'enroule sur ledit tambour afin de refouler le fluide sanguin, caractérisée par le fait que la coque extérieure (110) est essentiellement rigide et constitue un boîtier étanche dans lequel la poche fermée (120) peut se déformer, et par le fait que la poche fermée (120) est au moins partiellement réalisée en un matériau essentiellement inextensible dans deux directions, de façon que le volume intérieur d'origine de la poche (120) reste constant quelle que soit sa forme, ladite poche étant ainsi insensibilisée à une dépression extérieure tendant à augmenter son volume intérieur lors du refoulement du fluide sanguin.

2. Pompe sanguine selon la revendication 1, caractérisée par le fait que le matériau essentiellement inextensible est un composite constitué d'une couche de polymère (200) et de deux nappes croisées de fibres (201, 202) intimement liées au polymère.

3. Pompe sanguine selon la revendication 2, caractérisée par le fait que les deux nappes croisées de fibres (201, 202) sont noyées dans la couche de polymère (200).

4. Pompe sanguine selon la revendication 2, caractérisée par le fait que les deux nappes croisées de fibres (201, 202) constituent une trame collée extérieurement sur la surface (204) de la couche de polymère (200).

5. Pompe sanguine selon la revendication 2, caractérisée par le fait que les deux nappes croisées de fibres (201, 202) constituent une trame collée intérieurement sur la surface (205) de la couche de polymère (200).

6. Pompe sanguine selon l'une des revendications 2 à 5, caractérisée par le fait que les deux nappes croisées de fibres (201, 202) sont organisées selon deux directions sensiblement orthogonales.

7. Pompe sanguine selon l'une des revendications 2 à 6, caractérisée par le fait que les deux nappes croisées de fibres (201, 202) sont orientées diagonalement par rapport à une direction (Z) parallèle à la direction générale de l'axe (151) du tambour (150).

8. Pompe sanguine selon la revendication 7, caractérisée par le fait que les deux nappes croisées de fibres (201, 202) sont disposées symétriquement par rapport à ladite direction (Z) parallèle à l'axe (151) du tambour (150).

9. Pompe sanguine selon l'une des revendications 2 à 8, caractérisée par le fait que la couche de polymère (200) est réalisée en silicone ou en polyuréthane.

10. Pompe sanguine selon l'une des revendications 2 à 9, caractérisée par le fait que les fibres constitutives de l'une au moins des nappes croisées (201, 202) sont des fibres synthétiques.

11. Pompe sanguine selon l'une des revendications 2 à 9, caractérisée par le fait que les fibres constitutives de l'une au moins des nappes croisées (201, 202) sont des fibres de verre.

12. Pompe sanguine selon l'une des revendications 2 à 9, caractérisée par le fait que les fibres constitutives de l'une au moins des nappes croisées (201, 202) sont des fibres de carbone.

13. Pompe sanguine selon l'une des revendications 1 à 12, caractérisée par le fait que le matériau essentiellement inextensible concerne au moins la partie des parois (123, 124) de la poche (120) qui ne reste pas en contact avec la surface intérieure de la coque (110).

14. Pompe sanguine selon l'une des revendications 1 à 13, caractérisée par le fait que la poche (120) constitue un ensemble moulé en une seule pièce.

15. Pompe sanguine selon l'une des revendications 1 à 14, caractérisée par le fait que la coque extérieure essentiellement rigide (110) est réalisée à partir de fibres de carbone.

## Claims

1. An implantable blood pump (100) comprising an outer shell (110), at least one essentially flat and deformable closed bag (120) disposed inside the shell (110), having one of its walls (124) in contact with the inside surface of said shell, and being connected to an inlet valve (130) to enable it to be filled with blood fluid and to an outlet valve (140) to enable it to deliver blood fluid, a cylindrical drum (150) having an end edge (121) of the bag (120) fixed to a generator line thereof and organized to roll over the other wall (123) of said bag, a C-shaped bracket (160) mounted to rotate about the axis (151) of said drum and having its central portion connected by means of a flexible inextensible component (172) to the other end edge (122) of the bag (120), and an electric motor integrated within the drum (150) having its stator secured to said drum and having its rotor driving said bracket, said motor enabling the bracket (160) and the drum (150) to be driven in mutual and antagonistic rotation through a fraction of a turn to compress the bag (120) which winds onto said drum in order to expel the blood fluid, the pump being characterized by the fact that the outer shell (110) is essentially rigid and constitutes a sealed housing in which the closed bag (120) can deform, and by the fact that the closed bag (120) is made, at least in part, of a material that is essentially inextensible in two directions so that the original inside volume of the bag (120) remains constant regardless of its shape, said bag thus being made insensitive to external underpressure tending to increase its inside volume during blood fluid delivery.

2. A blood pump according to claim 1, characterized by the fact that the essentially inextensible material is a composite comprising a layer of polymer (200) and two crossed sheets of fibers (201, 202) that are intimately bonded with the polymer.

3. A blood pump according to claim 2, characterized by the fact that the two crossed sheets of fibers (201, 202) are embedded in the layer of polymer (200).

4. A blood pump according to claim 2, characterized by the fact that the two crossed sheets of fibers (201, 202) constitute a screen glued externally on the surface (204) of the polymer layer (200).

5. A blood pump according to claim 2, characterized by the fact that the two crossed sheets of fibers (201, 202) constitute a screen glued internally on the surface (205) of the layer of polymer (200).

6. A blood pump according to any one of claims 2 to 5, characterized by the fact that the two crossed sheets of fibers (201, 202) are organized in two substantially orthogonal directions.

7. A blood pump according to any one of claims 2 to 6, characterized by the fact that the two crossed sheets of fibers (201, 202) extend diagonally relative to a direction (Z) parallel to the general direction of the axis (151) of the drum (150).

8. A blood pump according to claim 7, characterized by the fact that the two crossed sheets of fibers (201, 202) are disposed symmetrically about said direction (Z) parallel to the axis (151) of the drum (150).

9. A blood pump according to any one of claims 2 to 8, characterized by the fact that the polymer layer (200) is made of silicone or of polyurethane.

10. A blood pump according to any one of claims 2 to 9, characterized by the fact that the fibers constituting at least one of the crossed sheets (201, 202) are synthetic fibers.

11. A blood pump according to any one of claims 2 to 9, characterized by the fact that the fibers constituting at least one of the crossed sheets (201, 202) are glass fibers.

12. A blood pump according to any one of claims 2 to 9, characterized by the fact that the fibers constituting at least one of the crossed sheets (201, 202) are carbon fibers.

13. A blood pump according to any one of claims 1 to 12, characterized by the fact that the essentially inextensible material occupies at least a portion of the walls (123, 124) of the bag (120) that does not remain in contact with the inside surface of the shell (110).

14. A blood pump according to any one of claims 1 to 13, characterized by the fact that the bag (120) constitutes a one-piece molded assembly.

15. A blood pump according to any one of claims 1 to 14, characterized by the fact that the essentially rigid outer shell (110) is made of carbon fibers.

## Patentansprüche

1. Implantierbare Blutpumpe (100) enthaltend eine äußere Schale (110), und mindestens einen weitgehend flachen und verformbaren Beutel (120), welcher in dieser Schale (110) mit einem seiner Wandteile (124) mit der Innenfläche dieser Schale in Berührung steht und an ein Einlaßventil (140) für dessen Befüllung mit der Blutflüssigkeit angeschlossen ist, und ein Auslaßventil (140) für den Ausstoß dieser Blutflüssigkeit, sowie eine zylindrische Trommel (150), auf deren einer Mantellinie eine Abschlußkante (121) des Beutels (120) befestigt ist und die so ausgelegt ist, daß sie auf der anderen Wand (123) dieses Beutels abgerollt werden kann, und einen C-förmigen Bügel (160), welcher drehbar auf der Achse (151) dieser Trommel gelagert ist und dessen zentraler Schenkel über ein elastisches nicht dehnbares Element (172) mit der anderen Abschlußkante (122) des Beutels (120) verbunden ist, und enthaltend einen in diese Trommel (150) integrierten elektrischen Motor, dessen Stator mit dieser Trommel fest verbunden ist und dessen Rotor diesen Bügel antreibt, wobei es dieser Motor ermöglicht, den Bügel (160) und die Trommel (150) im Rahmen einer teilweisen Umdrehung in einer relativen und antagonistischen Bewegung anzutreiben, um diesen Beutel (120) zu komprimieren, welcher auf diese Trommel aufgerollt wird, um die Blutflüssigkeit auszustoßen, **dadurch gekennzeichnet, daß** diese äußere Schale (110) weitgehend steif ist und ein abdichtendes Gehäuse bildet, in dem sich der geschlossene Beutel (120) verformen kann, und dadurch, daß der geschlossene Beutel (120) mindestens teilweise aus einem weitgehend in zwei Richtungen nicht dehnbaren Material hergestellt ist, damit das ursprüngliche Innenvolumen des Beutels (120) unabhängig von seiner Form konstant bleiben kann, wobei dieser Beutel ebenfalls gegen einen äußeren Unterdruck unempfindlich bleibt, welcher dazu geeignet ist, sein Innenvolumen während dem Ausstoß der Blutflüssigkeit zu erhöhen.

2. Implantierbare Blutpumpe nach Anspruch 1, **dadurch gekennzeichnet, daß**, das weitgehend nicht dehnbare Material ein Verbundwerkstoff ist, welcher aus einer Polymerschicht (200) und zwei gekreuzten Faserlagen (201, 202) besteht, die mit dem Polymer innig verbunden sind.

3. Implantierbare Blutpumpe nach Anspruch 2, **dadurch gekennzeichnet, daß** diese beiden gekreuzten Faserlagen (201, 202) in diese Polymerschicht (200) eingebettet sind.

4. Implantierbare Blutpumpe nach Anspruch 2, **dadurch gekennzeichnet, daß** diese beiden gekreuzten Faserlagen (201, 202) einen Raster bilden, welcher von der Außenseite an der Oberfläche (205) der Polymerschicht (200) verklebt ist.

5. Implantierbare Blutpumpe nach Anspruch 2, **dadurch gekennzeichnet, daß** diese beiden gekreuzten Faserlagen (201, 202) einen Raster bilden, welcher von der Innenseite der Oberfläche (205) der Polymerschicht (200) verklebt ist.

6. Implantierbare Blutpumpe nach einem der Ansprüche 2 bis 5, **dadurch gekennzeichnet, daß** diese beiden gekreuzten Faserlagen (201, 202) in zwei weitgehend rechtwinklig zueinander verlaufenden Richtungen ausgerichtet sind.

7. Implantierbare Blutpumpe nach einem der Ansprüche 2 bis 6, **dadurch gekennzeichnet, daß** diese beiden gekreuzten Faserlagen (201, 202) diagonal zu einer Richtung (2) ausgerichtet sind, welche parallel zu der allgemeinen Verlaufsrichtung der Achse (151) der Trommel (150) verläuft.

8. Implantierbare Blutpumpe nach Anspruch 7, **dadurch gekennzeichnet, daß** diese beiden gekreuzten Faserlagen (201, 202) symmetrisch zu der Richtung (2) parallel zur Achse (151) der Trommel (150) ausgerichtet sind.

9. Implantierbare Blutpumpe nach einem der Ansprüche 2 bis 8, **dadurch gekennzeichnet, daß** diese Polymerschicht (200) aus Silikon oder Polyurethan hergestellt ist.

10. Implantierbare Blutpumpe nach einem der Ansprüche 2 bis 9, **dadurch gekennzeichnet, daß** die mindestens eine der gekreuzten Faserlagen (201, 202) bildenden Fasern aus synthetische Fasern bestehen.

11. Implantierbare Blutpumpe nach einem der Ansprüche 2 bis 9, **dadurch gekennzeichnet, daß** die mindestens eine der gekreuzten Faserlagen (201, 202) bildenden Fasern aus Glasfasern bestehen.

12. Implantierbare Blutpumpe nach einem der Ansprüche 2 bis 9, **dadurch gekennzeichnet, daß** die mindestens eine der gekreuzten Faserlagen (201, 202) bildenden Fasern aus Kohlenstoffasern bestehen.

13. Implantierbare Blutpumpe flach einem der Ansprüche 1 bis 12, **dadurch gekennzeichnet, daß** das weitgehend nicht dehnbare Material mindestens in einen Teil der Wände (123, 124) des Beutels (120) eingebracht ist, welcher nicht mit der Innenfläche der Schale (110) in Berührung steht.

**14.** Implantierbare Blutpumpe nach einem der Ansprüche 1 bis 13,
**dadurch gekennzeichnet, daß**
der Beutel (120) aus einem in einem Stück gegossenen Element besteht.

**15.** Implantierbare Blutpumpe nach einem der Ansprüche 1 bis 14,
**dadurch gekennzeichnet, daß**
die weitgehend steife äußere Schale (110) aus Kohlenstoffasern hergestellt ist.

FIG_1

FIG_2

FIG_3

## FIG.4a

## FIG.4b

## FIG.4c

## FIG.5

## FIG.6

## FIG.7

## FIG.8